# EUROPEAN PATENT SPECIFICATION

(11) **EP 4 338 728 B1**
(45) Date of publication and mention of the grant of the patent: **30.09.2026**
(21) Application number: 23190459.0
(22) Date of filing: 09.08.2023
(51) Int. Cl.: A61K 9/08, A61K 31/7048, A61K 47/10, A61P 25/06, A61P 25/08

(54) **TOPIRAMATE LIQUID COMPOSITION AND ITS USE, PROCESS TO MANUFACTURE A COMPOSITION AND KIT**
FLÜSSIGE TOPIRAMATZUSAMMENSETZUNG UND IHRE VERWENDUNG, VERFAHREN ZUR HERSTELLUNG EINER ZUSAMMENSETZUNG UND KIT
COMPOSITION LIQUIDE DE TOPIRAMATE ET SON UTILISATION, PROCÉDÉ DE FABRICATION D'UNE COMPOSITION ET KIT

(30) Priority: 10.08.2022 BR 102022015798
(43) Date of publication of application: 20.03.2024
(73) Proprietor: EMS S.A., CEP-13186-901 Hortolandia, SP (BR)
(72) Inventor: LOUZA DE OLIVEIRA, Rodrigo, CEP 13186-901 São Paulo (BR); SANTOS, Fernando Henrique, CEP 13186-901 São Paulo (BR); RIBEIRO CATELLI, Ettamyr Eduardo, CEP 13186-901 São Paulo (BR)
(74) Representative: Oetke, Cornelia

(56) References cited:
- WO-A2-2020/053662
- US-A1- 2021 169 844

## Description

### TECHNICAL FIELD

This invention belongs to the pharmaceutical field and relates to liquid compositions of topiramate or a pharmaceutically acceptable salt thereof, uses thereof, manufacture processes, kits for preventing and/or treating epilepsy, convulsion, Lennox-Gastaut syndrome or migraine.

### BACKGROUND OF THE INVENTION

Epilepsy is conceptualized as a brain disorder characterized by a lasting predisposition to epileptic seizures, and by the neurobiological, social, cognitive and psychological consequences of this condition. The definition of epilepsy requires the occurrence of at least one epileptic seizure. Among the possible causes for its appearance, complications during childbirth, brain injuries, infection (for example, meningitis) and use of alcoholic beverages and/or drugs can be mentioned.

A seizure can be defined as an involuntary muscle contraction, causing disordered movements. It is usually accompanied by loss of consciousness. Seizures happen when the outer layer of the brain is excited. The seizure can be classified as a type of epileptic seizure.

Lennox-Gastaut syndrome (SLG) is considered an epileptic encephalopathy and is defined by a triad: 1) multiple drug-resistant seizure types, 2) a specific EEG pattern showing slow spike-wave complexes or generalized paroxysmal rapid activity, and 3) intellectual disability.

Migraine is a neurological, genetic and chronic disease whose main characteristic is throbbing headache, on one or both sides of the head, commonly accompanied by nausea, vomiting and intolerance to sounds, light, and strong smells.

In this context, topiramate was identified as an efficient therapeutic agent as it influences several chemical processes in the brain, reducing the excess excitability of nerve cells, which can trigger one or more of the selected diseases or disorders of seizure, epilepsy, and migraine.

Topiramate, IUPAC name [(1R,2S,6S,9R)-4,4,11,11-tetramethyl-3,5,7,10,12-pentaoxatricyclo[7.3.0.0²,⁶]dodecan-6-yl]methyl sulfamate, is a monosaccharide substituted with a sulfamate portion conceived initially as part of efforts to generate fructose-1,6-diphosphate analogues to block gluconeogenesis, in which topiramate was a synthetic intermediate. Through studies of its capacity as an anticonvulsant agent, the compound was shown to be highly active against maximal electroshock seizure and to have a long duration of action.

Structurally, topiramate does not resemble a normal therapeutic agent, as it has a very high concentration of oxygen. Topiramate has been approved in Europe since 2003, in the UK in 1956 and in the US since 2004. The appearance of the active substance is a white to almost white powder, soluble in alkaline solutions containing sodium hydroxide or sodium phosphate and having a pH of 9-10. It is freely soluble in acetone, chloroform, dimethylsulfoxide and ethanol and substantially insoluble in water.

Topiramate influences several chemical processes in the brain by reducing the over-excitability of nerve cells, which can cause epileptic seizures and migraine attacks. For treatment in patients with newly diagnosed epilepsy who are taking only topiramate or who will be switched to taking only topiramate, the therapeutic effect has been observed within two weeks of treatment. In therapy associated with other drugs in adults and children with partial or generalized tonic-clonic seizures, the therapeutic effect was observed in the first four weeks of treatment. For the prevention of migraine in adults, the therapeutic effect was observed within the first month after starting treatment.

Absorption of topiramate is rapid, with peak plasma concentrations occurring approximately 2 hours after a 400 mg oral dose. The relative bioavailability of topiramate from the tablet formulation is about 80% compared to a solution. Bioavailability is not affected by food.

There is evidence of its action as a mood stabilizer and as a neuroprotector. However, the precise mechanism by which topiramate exerts its anticonvulsant and migraine prophylactic effects has not yet been fully elucidated. It is known, however, that it blocks voltage-gated sodium channels, which probably leads to the control of sustained depolarizations during seizures. It also reduces membrane depolarization by AMPA/Kainate receptors and increases GABA(A) receptor activity, which enhances its inhibitory effects. Furthermore, topiramate is a weak inhibitor of carbonic anhydrase, and acidosis in the brain partially protects against seizures by regulating negatively the NMDA receptor activity.

There are solid compositions of topiramate on the market, in particular in the form of regular or extended-release tablets. Topamax^{®} 100mg, manufactured by Janssen Cilag, is in coated tablet format and is on ANVISA's reference drug list for that concentration (100mg).

At the time of this disclosure, there are no liquid topiramate compositions approved for commercialization in Brazil.

Compositions containing topiramate have additionally been disclosed in patent documents. For example, GB2594242 describes liquid topiramate compositions. However, as can be readily identified from GB2594242, topiramate concentrations range from 5 mg/ml to 10 mg/ml (see, for example, the abstract of said document).

It is important to emphasize in this regard that the substantial increase in the concentration of topiramate significantly alters the formulation of the composition in order to maintain the proper solubilization and stability of the active ingredient during and after preparing the liquid composition process, and in order to guarantee greater accuracy and speed of dosing, in addition to proper adherence to treatment.

Until the moment of this disclosure, there is no document or product based on topiramate or a pharmaceutically acceptable salt thereof with the liquid pharmaceutical form and concentration range claimed herein, overcoming the deficiencies identified in the prior art. WO 2020/053662 A2 discloses a non-aqueous solution, comprising an active pharmaceutical ingredient present at about 0.01 wt% to about 30 wt%, such as topiramate, and a solubilizer, e.g., propylene glycol, a surfactant and a vehicle at certain percentages. The composition can further comprise sweetening agent(s), i.e. sucralose, and flavoring agent(s). It discloses said pharmaceutical composition for treating a disease or disorder such as seizures (convulsive or nonconvulsive), epilepsy, Lennox-Gastaut sydrome, migraine; and a method of preparing said formulation. US 2021/169844 A1 discloses liquid pharmaceutical compositions comprising topiramate in an amount of from 1.2% to 10% (i.e., 100 mg/ml) and a solvent component comprising organic solvents, e.g. glycerin and polyethylene glycol at certain percentages; wherein the composition is substantially anhydrous. The composition can further comprise sweetener(s) {sucralose and xylitol mentioned in long list} and flavorant(s)). Said composition is for treating conditions and disorders including epilepsy, seizures, seizures associated with Lennox-Gastaut syndrome and migraine headaches.

In this context, it is advantageous to develop new topiramate composition alternatives that can be more easily administered, with dosage accuracy, and that have adequate pharmacological action. Therefore, this invention relates to new and inventive liquid compositions of topiramate or a pharmaceutically acceptable salt thereof.

### SUMMARY OF THE INVENTION

This invention discloses new compositions of topiramate or a pharmaceutically acceptable salt thereof, their uses, preparation processes and related kits. In particular, the pharmaceutically active ingredient topiramate has the following structural formula:

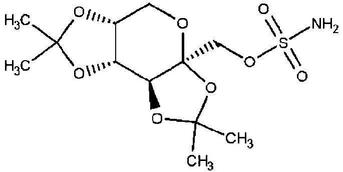

As indicated herein, the prior art already discloses topiramate compositions. However, it fails to reveal or suggest the necessary teachings to achieve the main object of this invention, that is, a liquid composition of topiramate or a pharmaceutically acceptable salt thereof substantially free of water at a high concentration while maintaining organoleptic characteristics at acceptable levels.

In particular, despite the known low solubility and difficulty of formulating the active ingredient topiramate and its strong bitter taste in solution, the inventors of this invention were unexpectedly able to identify excipients and concentration ranges that make it possible to obtain a composition with a high concentration of topiramate or a pharmaceutically acceptable salt thereof that is stable and can be administered to a patient in need, assuring accuracy and speed of dosing and adequate adherence to treatment of the disorder or disease selected from epilepsy, seizure or migraine.

Preferably, the composition of this invention is water-free. Furthermore, the composition may be free of preservatives.

### DETAILED DESCRIPTION OF THE INVENTION

The invention is set out in the appended set of claims.

Novel liquid compositions of topiramate or a pharmaceutically acceptable salt thereof substantially free of water, their uses, preparation processes and related kits are disclosed. According to this invention, the concentration of topiramate or a pharmaceutically acceptable salt thereof in the liquid composition can be selected from the range of 50 to 150 mg/ml. Preferably, the concentration of topiramate or a pharmaceutically acceptable salt thereof in the liquid composition is selected from the range of 75 to 125 mg/ml. Even more preferably, the concentration of topiramate or a pharmaceutically acceptable salt thereof in the liquid composition is 100 mg/ml.

When compared to the tablet pharmaceutical form, the liquid composition has the advantage of being easier to use, through improved administration and swallowing. Particularly for the pediatric population, the introduction of the liquid pharmaceutical form of topiramate will make it easier and faster to calculate the daily dose. In this sense, the calculation of the dosage based on kilograms can result in varied dosages, which can cause significant errors due to the need to split the tablet and, consequently, in the dosage actually administered. Ultimately, such errors may compromise patient safety in administering the composition.

Still, even considering liquid compositions of topiramate, those skilled in the art face substantial difficulties in solubilizing the active ingredient, keeping it stable in the composition during and after the preparation process. In general, it is understood that the formulation difficulty increases proportionally to the increase in topiramate concentration, thus demanding inventive efforts from the inventors.

The liquid composition of this invention overcomes the deficiencies of the prior art related to solid topiramate compositions (tablets, capsules and others), since it substantially mitigates, in its application, dosage errors with the dosage in drops or other suitable release mechanism.

Additionally, this invention cannot be considered obvious in view of previously disclosed liquid compositions since it would not be possible to scale the formulation of the composition with a significant increase in the concentration of topiramate, maintaining acceptable organoleptic properties for the patient despite the strongly bitter character. of the active ingredient, which is potentiated by increasing its concentration, ensuring accuracy and speed of dosing and adequate adherence to the treatment of the disorder or disease selected from among epilepsy, seizures or migraine.

At least for these reasons, the compositions of this invention guarantee greater therapeutic efficacy and patient adherence to their use.

In a first embodiment, this invention features a composition comprising a therapeutically effective amount of topiramate or a pharmaceutically acceptable salt thereof, and one or more pharmaceutically acceptable excipients.

As described herein, topiramate or a pharmaceutically acceptable salt thereof may be in amorphous form or any stable crystalline form, considering the polymorphism of the molecule. Preferably, topiramate or a pharmaceutically acceptable salt thereof is in crystalline form I.

According to this invention, a pharmaceutically acceptable excipient is defined as any substance, other than the active pharmaceutical ingredient, that has been assessed for its safety and/or is approved for pharmaceutical use by a competent regulatory body, if applicable. Such excipients are selected according to the pharmaceutical dosage form of interest, its route of administration, physical-chemical compatibility with the active ingredient and the effect on efficacy. For example, pharmaceutically acceptable excipients can be selected from ROWE, R.C. et al. "Handbook of Pharmaceutical Excipients", 6th ed. USA, 2009, the Brazilian Pharmacopoeia or foreign correspondent, such as the American Pharmacopoeia or the European Pharmacopoeia.

According to this invention, the one or more pharmaceutically acceptable excipients can be selected from the group consisting of preservatives, sweeteners, thickeners, stabilizers, flavoring agents, chelators, solvents, solubilizers, and the same excipient can have more than one function in the composition. In some embodiments of the invention, the composition comprises solvents, solubilizers, sweeteners and flavoring excipients. In preferred embodiments of this invention, the composition does not need a preservative in its formulation due to the fact that it is water-free.

According to this invention, the solvent may be selected from the group consisting of mono-, di-, or trihydroxy alcohols or combinations thereof. In preferred embodiments, the solvent is selected from glycerol, propylene glycol and combinations thereof.

According to this invention, the solubilizer may be selected from the group consisting of lecithin, macrogol, polyethylene glycol, poloxamer or combinations thereof. In preferred embodiments, the solubilizer is macrogol.

According to this invention, the sweetener can be selected from the group consisting of natural or synthetic sweeteners, or combinations thereof, which impart a sweet taste to the composition. In preferred embodiments, the sweetener is selected from neohesperidin dihydrochalcone, steviol glycosides, mannitol, sorbitol, sucralose, thaumatin, xylitol and combinations thereof. In even more preferred embodiments, the sweetener is selected from neohesperidin dihydrochalcone, sucralose, xylitol and combinations thereof.

According to this invention, the flavoring can be selected from the group consisting of any agents capable of masking unpleasant tastes present in the composition, including natural and artificial flavorings. In preferred embodiments, the flavoring is selected from the group consisting of cherry essence, raspberry essence, orange essence, mint essence, strawberry essence, chocolate essence or combinations thereof. In even more preferred embodiments, the flavoring is selected from raspberry essence, mint essence and combinations thereof.

As previously mentioned, until the moment of this disclosure, no pharmaceutical form for topiramate or a pharmaceutically acceptable salt thereof in the form of administration and with the concentration hereby disclosed had been taught or even suggested. In this regard, the inventors of this application focused on the development of new pharmaceutical forms of topiramate or a pharmaceutically acceptable salt thereof that maintained its pharmacological action, while allowing adequate patient comfort and adherence and precise control of the composition dosage.

When developing the new compositions, the solubilization and stabilization of topiramate or a pharmaceutically acceptable salt thereof in liquid medium proved to be challenging considering the extremely low solubility of the compound in aqueous solution at room temperature. Thus, the inventors prepared several combinations in order to identify optimized concentration ranges for the components of the composition, that is, topiramate or a pharmaceutically acceptable salt thereof associated with one or more excipients. Such preliminary tests indicated that using water in the solvent/solubilizer system negatively affected the composition solubility.

Thus, several alternatives of pharmaceutically acceptable systems were tested that would allow the complete dissolution of topiramate or a pharmaceutically acceptable salt thereof and homogenization of the composition during its preparation process, even considering the high target concentration for the active ingredient.

Combinations comprising one or more of mono-, di-, or trihydroxy alcohols, lecithin, macrogol, polyethylene glycol, and poloxamer were tested. Surprisingly, the inventors found that combinations of these compounds in certain concentration ranges were particularly suited to the ready homogenization of topiramate or a pharmaceutically acceptable salt during the process of forming compositions with the active ingredient present in high concentrations. It was further noted that the formulation of compositions according to this invention enabled a stabilization of topiramate or a pharmaceutically acceptable salt thereof in solution, without reformation of solids.

In some embodiments of the invention, the final concentration of topiramate in the composition can range from 50 to 150 mg/ml of the composition. In preferred embodiments, the final concentration of topiramate in the composition can range from 75 to 125 mg/ml of the composition. In further preferred embodiments, the final concentration of topiramate in the composition is 100 mg/ml of the composition.

In some embodiments of the invention, the final concentration of solvent in the composition can range from 40 to 70% w/w of the composition and the final concentration of solubilizer in the composition can range from 20 to 40% w/w of the composition. In preferred embodiments, the final concentration of solvent in the composition can range from 45 to 65% w/w of the composition and the final concentration of solubilizer in the composition can range from 25 to 35% w/w of the composition. In other preferred embodiments, the solvent/solubilizer system comprises one or more alcohols and macrogol. In further preferred embodiments, the solvent/solubilizer system comprises glycerol at approximately 37.7 ± 0.5% w/w, propylene glycol at approximately 18.1 ± 0.5% w/w, and macrogol 400 at approximately 30.8 ± 0.5% w/w.

Another particularly advantageous factor of this invention is that, due to the absence of water in the manufacturing process of the composition, it does not need a preservative.

Otherwise, the high percentage of non-aqueous solvents associated with the presence of topiramate significantly affect the organoleptic properties of the composition. In particular, this association gives a strongly bitter taste to the product. Therefore, in order to ensure an adequate patient experience with the administration of the liquid composition, ensuring better adherence to the use of the composition, it is necessary to add compounds that mask the composition taste. In this regard, the composition of this invention additionally comprises sweeteners and flavoring agents, which may be natural or artificial. In preferred embodiments, the sweeteners used are neohesperidin, sucralose and xylitol and the flavoring agents are mint essence and raspberry essence. This combination of sweeteners and flavoring agents is capable of adequately masking the original taste of the compositions of this invention, even with high concentrations of topiramate.

In a second embodiment, this invention features the use of the topiramate liquid composition for preparing a drug for preventing and/or treating convulsion, epilepsy or migraine.

Compositions of this invention can be suitably administered in a therapeutically effective amount to patients in need for prevention or treatment of seizure, epilepsy or migraine. Preferably, the subject is human. Further, in some preferred embodiments, administration of the liquid topiramate composition is selected from the group comprising oral, sublingual, nasal, parenteral, injectable, submuscular, topical, and transdermal routes. In even more preferred embodiments, administration of the liquid topiramate composition is orally.

In a third embodiment, this invention describes processes for preparing a liquid topiramate composition comprising the following steps:
a) in a main reactor, mix a quantity of solvent(s) and solubilizer(s) under stirring at a temperature between 25 and 50°C and stirring speed of 300 to 700 rpm until the system is homogenized;
b) increase the stirring speed to 800 to 1,200 rpm, adding topiramate, maintaining the stirring for 20 to 40 minutes until the system is homogenized;
c) increase the agitation speed to 1300 to 1700 rpm, adding the sweetener(s), maintaining the stirring for 40 to 80 minutes until the system is homogenized;
d) in an auxiliary reactor, mix under stirring a quantity of solvent(s) and solubilizer(s) and flavor(s) at a stirring speed of 100 to 500 rpm for 5 to 15 minutes until homogenization of the system;
e) in the main reactor, containing the mixture of item c), add the mixture of item d) at a temperature between 20 and 40°C and stirring speed of 1,300 to 1,700 rpm for 5 to 15 minutes until the system is homogenized;
f) add an additional quantity of solvent(s) to the system to reach the target concentration of topiramate, maintaining the stirring speed of item e) for 20 to 40 minutes until the system is homogenized; and
g) optionally, bottling the generated composition using appropriate bottling parameters and equipment, considering the volume of the final product.

As will be evident, several modifications of parameters, such as temperature, speed and stirring time, can be carried out in the process depending on the excipient system chosen, without, however, departing from the general process disclosed herein.

In a fourth embodiment, this invention provides a kit comprising the disclosed liquid topiramate composition. The kit according to this invention can comprise such a composition associated with any suitable delivery device. Delivery devices commonly used in the pharmaceutical field include, without limitation, drippers, ampoules, syringes and others.

### EXAMPLES:

The examples below, described in detail, serve to illustrate embodiments of this invention without having, however, a limiting character to its scope of protection.

### Example 1 - Process of Preparing a Liquid Composition of Topiramate

The following is a process for preparing a liquid topiramate composition.

In a main reactor, glycerol, macrogol and propylene glycol are mixed at a temperature between 35-45°C at a stirring speed of 500 rpm until homogenization. Next, the stirring speed is increased to 1,000 rpm with the addition of topiramate for 30 minutes to homogenize the active ingredient. Subsequently, the stirring speed is increased to 1,500 rpm with the addition of neohesperidin dihydrochalcone, sucralose and xylitol for 60 minutes to homogenize these excipients.

At the same time, in an auxiliary reactor, propylene glycol, mint essence and raspberry essence are mixed at a stirring speed of 300 rpm for 10 minutes until these excipients are homogenized.

The mixture resulting from the auxiliary reactor is added to the main reactor at a temperature between 25-30°C at a stirring speed of 1,500 rpm for 10 minutes to homogenize the components. Next, an additional quantity of glycerol is added to the main reactor to reach the target concentration of topiramate, keeping the system under stirring at 1,500 rpm for 30 minutes until the final liquid formulation is homogenized.

### Example 2 - Exemplary Liquid Composition

The following is an exemplary liquid composition of topiramate at 100 mg/ml solution prepared according to the procedure of Example 1. Table 1 indicates each of the raw materials, quantities, proportions and respective functions in the exemplary formulation.

**Table 1: Exemplary Composition**

| **Raw Material** | **Quantity (mg/ml)** | | **% w/w in pharmaceutical form** | | **Function in the formula** |
|---|---|---|---|---|---|
| Topiramate | 100.000 | | 8.230 | | Active ingredient |
| Glycerol | 458.070 | | 37.701 | | Solvent |
| Macrogol | 375.000 | | 30.864 | | Solubilizer |
| Propylene glycol | 220.000 | | 18.107 | | Solvent |
| Neohesperidin dihydrochalcone | 0.506 | | 0.042 | | Sweetener |
| Sucralose | 30.011 | | 2.470 | | Sweetener |
| Xylitol | 30.011 | | 2.470 | | Sweetener |
| Mint essence | 0.902 | | 0.074 | | Flavoring |
| Raspberry Essence | 0.506 | | 0.042 | | Flavoring |

It should be understood that the embodiments described above are merely illustrative and that various modifications can be made to them by a person skilled in the art without departing from the scope of this invention. Consequently, this invention should not be considered limited to the exemplary embodiments described in this application. Furthermore, this disclosure may include subject matter not currently claimed, but which may be claimed in the future in combination with or separately from the features claimed herein.

## Claims

1. Non-aqueous liquid composition **characterized in that** it comprises topiramate or a pharmaceutically acceptable salt thereof at a concentration of 100 mg/ml associated with excipients and/or pharmaceutically acceptable vehicles, wherein the excipients comprise:
a) solvents selected from the group consisting of glycerol, propylene glycol and combinations thereof at 40 to 70% w/w of the composition;
b) a solubilizer consisting of macrogol at 20 to 40% w/w of the composition;
c) sweeteners consisting of neohesperidin dihydrochalcone, sucralose and xylitol; and
d) flavors consisting of raspberry essence and mint essence.

2. Liquid composition according to claim 1, **characterized in that** (a) it is in the form of a solution or an emulsion, preferably a solution; and/or (b) it does not comprise a preservative.

3. Liquid composition according to claim 1 or claim 2, **characterized in that** the solvents are present in a concentration of about 55 to 65% w/w and the solubilizer is present at a concentration of 25 to 35%.

4. The liquid composition according to any one of claims 1 to 3 for use in therapy.

5. The liquid composition according to any one of claims 1 to 3 for use in preventing or treating a disease or disorder selected from epilepsy, convulsion, syndrome of Lennox-Gastaut or migraine.

6. Process to manufacture a liquid composition of topiramate or a pharmaceutically acceptable salt thereof **characterized by** comprising the steps of:
a) mixing under stirring an amount of one or more solvents and a solubilizer at a temperature between 25 and 50°C and stirring speed of 300 to 700 rpm until the system is homogenized;
b) increasing the stirring speed to 800 to 1,200 rpm, adding topiramate or a pharmaceutically acceptable salt thereof, maintaining stirring for 20 to 40 minutes until the system is homogenized;
c) increasing the stirring speed to 1,300 to 1,700 rpm, adding one or more sweeteners, maintaining the stirring for 40 to 80 minutes until the system is homogenized;
d) separately, mixing under stirring a quantity of one or more solvents and a solubilizer and one or more flavors at a stirring speed of 100 to 500 rpm for 5 to 15 minutes until the system is homogenized;
e) in the main reactor, containing the mixture of item c), adding the mixture of item d) at a temperature between 20 and 40°C and stirring speed of 1,300 to 1,700 rpm for 5 to 15 minutes until the system is homogenized;
f) adding additional quantity of solvent(s) to the system to reach the target concentration of topiramate by maintaining the stirring speed of item e) for 20 to 40 minutes until the system is homogenized
wherein the one or more solvents are selected from the group consisting of glycerol, propylene glycol and combinations thereof;
wherein the solubilizer is macrogol;
wherein the one or more sweeteners are neohesperidin dihydrochalcone, sucralose and xylitol;
wherein the one or more flavors are raspberry essence and mint essence; and wherein the target concentration of topiramate or a pharmaceutically acceptable salt thereof is 100 mg/ml and wherein the final concentration of solvents in the composition ranges from 40% to 70% w/w and the final concentration of solubilizers in the composition ranges from 20 to 40% w/w.

7. Process according to claim 6, **characterized in that** it additionally comprises an additional step:
g) bottling the generated composition using appropriate bottling parameters and equipment, considering the volume of the final product.

8. Kit **characterized by** comprising a system comprising the liquid composition of topiramate, as defined in claim 1, a release device and optionally administration instructions.

9. Kit, according to claim 8, **characterized in that** the release device is a calibrated dripper for standardizing the volume of the liquid formulation.

## Patentansprüche

1. Nichtwässrige flüssige Zusammensetzung, **dadurch gekennzeichnet, dass** sie Topiramat oder ein pharmazeutisch verträgliches Salz davon in einer Konzentration von 100 mg/ml in Verbindung mit Hilfsstoffen und/oder pharmazeutisch verträglichen Trägern enthält, wobei die Hilfsstoffe Folgendes umfassen:
a) Lösungsmittel, ausgewählt aus der Gruppe bestehend aus Glycerin, Propylenglykol und Kombinationen davon, in einer Menge von 40 bis 70 Gew.-% der Zusammensetzung;
b) einen Lösungsvermittler, bestehend aus Macrogol, in einer Menge von 20 bis 40 Gew.-% der Zusammensetzung;
c) Süßstoffe, bestehend aus Neohesperidin-Dihydrochalkon, Sucralose und Xylit; und
d) Aromastoffe, bestehend aus Himbeer- und Minz-Essenz.

2. Flüssige Zusammensetzung nach Anspruch 1, **dadurch gekennzeichnet, dass** (a) sie in Form einer Lösung oder einer Emulsion, vorzugsweise einer Lösung, vorliegt; und/oder (b) sie kein Konservierungsmittel enthält.

3. Flüssige Zusammensetzung nach Anspruch 1 oder Anspruch 2, **dadurch gekennzeichnet, dass** die Lösungsmittel in einer Konzentration von etwa 55 bis 65 Gew.-% und der Lösungsvermittler in einer Konzentration von 25 bis 35 % vorliegen.

4. Die flüssige Zusammensetzung gemäß einem der Ansprüche 1 bis 3 zur Verwendung in der Therapie.

5. Die flüssige Zusammensetzung gemäß einem der Ansprüche 1 bis 3 zur Verwendung bei der Vorbeugung oder Behandlung einer Krankheit oder Störung, ausgewählt aus Epilepsie, Krampfanfällen, dem Lennox-Gastaut-Syndrom oder Migräne.

6. Verfahren zur Herstellung einer flüssigen Zusammensetzung von Topiramat oder eines pharmazeutisch verträglichen Salzes davon, **gekennzeichnet durch** die folgenden Schritte:
a) Mischen einer Menge eines oder mehrerer Lösungsmittel und eines Lösungsvermittlers unter Rühren bei einer Temperatur zwischen 25 und 50 °C und einer Rührgeschwindigkeit von 300 bis 700 Umdrehungen pro Minute (U/min), bis das System homogenisiert ist;
b) Erhöhen der Rührgeschwindigkeit auf 800 bis 1.200 U/min, Zugabe von Topiramat oder eines pharmazeutisch verträglichen Salzes davon, Aufrechterhalten des Rührens für 20 bis 40 Minuten, bis das System homogenisiert ist;
c) Erhöhen der Rührgeschwindigkeit auf 1.300 bis 1.700 U/min, Zugabe eines oder mehrerer Süßungsmittel, Aufrechterhalten des Rührens für 40 bis 80 Minuten, bis das System homogenisiert ist;
d) separates Mischen unter Rühren von einer Menge eines oder mehrerer Lösungsmittel und eines Lösungsvermittlers sowie eines oder mehrerer Aromastoffe bei einer Rührgeschwindigkeit von 100 bis 500 U/min für 5 bis 15 Minuten, bis das System homogenisiert ist;
e) Zugabe der Mischung gemäß d) zu der im Hauptreaktor befindlichen Mischung gemäß c) bei einer Temperatur zwischen 20 und 40 °C und einer Rührgeschwindigkeit von 1.300 bis 1.700 U/min, für 5 bis 15 Minuten, bis das System homogenisiert ist;
f) Zugabe einer zusätzlichen Menge an Lösungsmittel(n) zu dem System, um die Zielkonzentration an Topiramat zu erreichen, wobei die Rührgeschwindigkeit aus Schritt e) 20 bis 40 Minuten lang beibehalten wird, bis das System homogenisiert ist,
wobei das eine oder die mehreren Lösungsmittel ausgewählt sind aus der Gruppe bestehend aus Glycerin, Propylenglykol und Kombinationen davon;
wobei der Lösungsvermittler Macrogol ist;
wobei der eine oder die mehreren Süßstoffe Neohesperidin-Dihydrochalkon, Sucralose und Xylit sind;
wobei es sich bei dem einen oder den mehreren Aromastoffen um Himbeer- und Minz-Essenz handelt; und
wobei die Zielkonzentration von Topiramat oder eines pharmazeutisch verträglichen Salzes davon 100 mg/ml beträgt und wobei die Endkonzentration der Lösungsmittel in der Zusammensetzung im Bereich von 40 % bis 70 % (Gew./Gew.) und die Endkonzentration der Lösungsvermittler in der Zusammensetzung im Bereich von 20 % bis 40 % (Gew./Gew.) liegt.

7. Verfahren nach Anspruch 6, **dadurch gekennzeichnet, dass** es zusätzlich einen weiteren Schritt umfasst:
g) Abfüllen der hergestellten Zusammensetzung unter Verwendung geeigneter Abfüllparameter und -anlagen unter Berücksichtigung des Volumens des Endprodukts.

8. Kit, **dadurch gekennzeichnet, dass** es ein System umfasst, das die in Anspruch 1 definierte flüssige Topiramat-Zusammensetzung, eine Abgabevorrichtung und gegebenenfalls eine Gebrauchsanweisung umfasst.

9. Kit nach Anspruch 8, **dadurch gekennzeichnet, dass** die Abgabevorrichtung ein kalibrierter Tropfer zur Standardisierung des Volumens der flüssigen Formulierung ist.

## Revendications

1. Composition liquide non aqueuse **caractérisée en ce qu'**elle comprend du topiramate ou un sel pharmaceutiquement acceptable de celui-ci à une concentration de 100 mg/mL associé à des excipients et/ou des véhicules pharmaceutiquement acceptables, dans laquelle les excipients comprennent :
a) des solvants choisis parmi le groupe constitué de glycérol, de propylène glycol et de combinaisons de ceux-ci à hauteur de 40 à 70 % p/p de la composition ;
b) un agent solubilisant consistant en du macrogol à hauteur de 20 à 40 % p/p de la composition ;
c) des édulcorants consistant en de la néohespéridine dihydrochalcone, du sucralose et du xylitol ; et
d) des arômes consistant en de l'essence de framboise et de l'essence de menthe.

2. Composition liquide selon la revendication 1, **caractérisée en ce que** (a) elle est sous la forme d'une solution ou d'une émulsion, préférentiellement une solution ; et/ou (b) elle ne comprend pas de conservateur.

3. Composition liquide selon la revendication 1 ou la revendication 2, **caractérisée en ce que** les solvants sont présents à une concentration d'environ 55 à 65 % p/p et l'agent solubilisant est présent à une concentration de 25 à 35 %.

4. Composition liquide selon l'une quelconque des revendications 1 à 3 pour son utilisation dans un traitement.

5. Composition liquide selon l'une quelconque des revendications 1 à 3 pour son utilisation dans la prévention ou le traitement d'une maladie ou d'un trouble choisi parmi l'épilepsie, la convulsion, le syndrome de Lennox-Gastaut ou la migraine.

6. Procédé de fabrication d'une composition liquide de topiramate ou d'un sel pharmaceutiquement acceptable de celui-ci, **caractérisé en ce qu'**il comprend les étapes suivantes :
a) le mélange sous agitation d'une quantité d'un ou de plusieurs solvants et d'un agent solubilisant à une température comprise entre 25 et 50 °C et à une vitesse d'agitation de 300 à 700 tours par minute jusqu'à homogénéisation du système ;
b) l'augmentation de la vitesse d'agitation à une valeur comprise entre 800 et 1 200 tours par minute, l'ajout de topiramate ou d'un sel pharmaceutiquement acceptable de celui-ci, le maintien de l'agitation pendant 20 à 40 minutes jusqu'à homogénéisation du système ;
c) l'augmentation de la vitesse d'agitation à une valeur comprise entre 1 300 et 1 700 tours par minute, l'ajout d'un ou de plusieurs édulcorants, le maintien de l'agitation pendant 40 à 80 minutes jusqu'à homogénéisation du système ;
d) séparément, le mélange sous agitation d'une quantité d'un ou de plusieurs solvants et d'un agent solubilisant et d'un ou de plusieurs arômes à une vitesse d'agitation de 100 à 500 tours par minute pendant 5 à 15 minutes jusqu'à homogénéisation du système ;
e) dans le réacteur principal, contenant le mélange du point c), l'ajout du mélange du point d) à une température comprise entre 20 et 40 °C et à une vitesse d'agitation de 1 300 à 1 700 tours par minute pendant 5 à 15 minutes jusqu'à homogénéisation du système ;
f) l'ajout d'une quantité supplémentaire de solvant(s) au système afin d'atteindre la concentration cible de topiramate en maintenant la vitesse d'agitation du point e) pendant 20 à 40 minutes jusqu'à homogénéisation du système ;
dans lequel les un ou plusieurs solvants sont choisis parmi le groupe constitué de glycérol, de propylène glycol et de combinaisons de ceux-ci ;
dans lequel l'agent solubilisant est le macrogol ;
dans lequel les un ou plusieurs édulcorants sont la néohespéridine dihydrochalcone, le sucralose et le xylitol ;
dans lequel les un ou plusieurs arômes sont l'essence de framboise et l'essence de menthe ; et
dans lequel la concentration cible de topiramate ou d'un sel pharmaceutiquement acceptable de celui-ci est de 100 mg/mL et dans lequel la concentration finale de solvants dans la composition est comprise entre 40 % et 70 % p/p et la concentration finale d'agents solubilisant dans la composition est comprise entre 20 et 40 % p/p.

7. Procédé selon la revendication 6, **caractérisé en ce qu'**il comprend en outre une étape supplémentaire :
g) le remplissage de la composition générée à l'aide de paramètres et d'équipement de remplissage appropriés, en tenant compte du volume du produit final.

8. Kit **caractérisé en ce qu'**il comprend un système comprenant la composition liquide de topiramate, telle que définie dans la revendication 1, un dispositif de distribution et éventuellement des instructions d'administration.

9. Kit selon la revendication 8, **caractérisé en ce que** le dispositif de distribution est un compte-gouttes étalonné pour obtenir le volume standard de la formulation liquide.
